Europäisches Patentamt

European Patent Office

Office européen des brevets

(19)

(11) Publication number: 0 142 059

A2

# (12) EUROPEAN PATENT APPLICATION

(21) Application number: 84112515.6

(22) Date of filing: 17.10.84

(51) Int. Cl.⁴: **A 61 L 2/04**
//A61K35/16

(30) Priority: 19.10.83 JP 196856/83

(43) Date of publication of application:
22.05.85 Bulletin 85/21

(84) Designated Contracting States:
BE DE FR GB NL SE

(71) Applicant: THE GREEN CROSS CORPORATION
15-1, Imabashi-1-chome Higashi-ku Osaka-shi
Osaka 541(JP)

(72) Inventor: Iga, Yoshiro
9-33, Danjocho 1-chome
Nishinomiya-shi Hyogo(JP)

(72) Inventor: Okano, Kanemichi
2-3-610, Minase 2-chome Shimamoto-cho
Mishima-gun Osaka(JP)

(72) Inventor: Akira, Toshiaki
19-3, Yamatadei 6-chome
Ibaraki-shi Osaka(JP)

(72) Inventor: Kameyama, Shoju
D15-505, Otokoyama Sasatani 5-banchi
Yawata-shi, Kyoto(JP)

(74) Representative: von Kreisler, Alek, Dipl.-Chem. et al,
Deichmannhaus am Hauptbahnhof
D-5000 Köln 1(DE)

(54) Method of heat-treating plasma proteins.

(57) The invention relates to a method of heat-treating heat-labile plasma proteins for virus inactivation comprising heating a virus-contaminated plasma protein in a dry state and in the presence of both an acidic amino acid and a basic amino acid until virus inactivation is complete.

EP 0 142 059 A2

Croydon Printing Company Ltd.

# METHOD OF HEAT-TREATING PLASMA PROTEINS

## FIELD OF INVENTION

This invention relates to a method of heat-treating heat-labile plasma proteins for virus inactivation.

## BACKGROUND OF INVENTION

The most trustworthy known method of inactivating viruses contained as foreign elements in plasma proteins such as albumin is the heat treatment in aqueous solution. This method (hereinafter referred to as "liquid-phase heat treatment") has been widely used for a long period of time since its adoption based on the report by Murray et al. [The New York Academy of Medicine, 31 (5), 341-358 (1955)]. The effectiveness of the liquid-phase heat treatment in virus inactivation has also been established epidemiologically.

However, only a few of plasma proteins, for instance albumin, can endure such liquid-phase heat treatment. Plasma proteins having physiologic or biologic activity are particularly sensitive to heat and easily undergo thermal denaturation, which likely results in reduction or loss in activity.

On the other hand, experiments using blood coagulation factor VIII as a model have revealed that heat treatment of plasma proteins in the water-free or substantially water-free dry condition (generally at a water content of not more than 3%, preferably not more than 1%) (hereinafter, "dry
heat treatment") can result in prevention, to a significant
extent, of loss in activity as compared with the liquid-
phase heat treatment (cf. Table 1).   However, even in the
dry heat treatment, plasma proteins cannot be free from
losses in activity without addition of stabilizing agents and,
furthermore, their solubility and the clarity of their solutions become worsened.

Meanwhile, it is said that the mechanism of virus inactivation upon heating in the liquid-phase is essentially
the denaturation of viral protein components but that, in
the case of dry heat treatment, the virus inactivation
mechanism principally involves the oxidative damage of
viral lipid components which leads to loss of pathogenicity.
Suggestedly, the virus inactivation mechanisms in both the
methods differ in substance from each other although they
partly overlap with each other [Rahn: Physical Methods of
Sterilization of Microorganisms, Bact. Rev., 9, 1-47 (1945)].

Under these circumstances, the present inventors conducted an intensive study in search of stabilizing agents
for plasma proteins in the dry heat treatment thereof and
a method of improving the dissolution behavior of plasma
proteins in water and found that, when the dry heat treatment of plasma proteins is conducted in the presence of
both an acidic amino acid and a basic amino acid, virus
inactivation can be attained under marked stabilization of

- 3 -    0142059

plasma proteins and that the plasma proteins heat-treated
in the dry state under such conditions are satisfactory in
solubility and dissolution behavior in water.  This finding
and additional studies have now led to completion of the
present invention.

SUMMARY OF INVENTION

Accordingly, a primary object of the invention is to
provide a method of heat-treating plasma proteins for virus
inactivation without impairing their original activity.

Another object of the invention is to provide a method
of heat-treating plasma proteins for virus inactivation
without impairing their solubility in water or the clarity
of their solution in water.

Thus, the present invention provides a method of heat-
treating plasma proteins which comprises heating a virus-
contaminated plasma protein in a dry state and in the presence of both an acidic amino acid and a basic amino acid
until virus inactivation is complete, whereby the stability
and the clarity of their solution in water of plasma proteins
are simultaneously improved.

DETAILED DESCRIPTION OF INVENTION

The plasma protein to be heat-treated in accordance
with the invention is a plasma protein fractionation product or a mixture of two or more plasma protein fractionation products and generally has biologic or physiologic activity. Examples of such plasma protein are blood coagulation factor II, factor V, factor VII, factor VIII, factor IX, factor X, factor XIII, fibrinogen, thrombin, plasminogen, albumin and globulin.

The acidic amino acid to be used in accordance with the invention includes, among others, aspartic acid and glutamic acid, and the basic amino acid includes, among others, histidine, lysine and arginine. These may be in the L, D or DL form but are preferably in the L form. Preferred examples of the combination of acidic amino acid and basic amino acid are the combination of lysine and glutamic acid, in particular the combination of L-lysine and L-glutamic acid, and the combination of lysine and aspartic acid.

The acidic amino acid and basic amino acid may be used in the form of a salt formed by them.

The acidic amino acid and basic amino acid are preferably used in a molar ratio of 0.5:1 to 2:1, more preferably 1:1.

Generally, the acidic amino acid and basic amino acid are used in a total amount of about 0.01-10% by weight based on the plasma protein. A stabilizing effect and an improvement in dissolution behavior in water (inclusive of clarity) can be produced already at an addition level of about 0.01% by weight, and the stabilization effect and improvement in solubility (clarity) reach saturation at an addition level of about 10% by weight. A preferred addition level is in the range of about 0.5-1% by weight. At an addition level in such range, the process of making preparations is best balanced with the stabilizing effect and improvement in solubility (clarity). The above addition level is applicable not only to the case in which both the amino acids are used as a composition but also to the case in which both the amino acids are used in the form of a salt composed thereof.

Both the amino acids may be added either before lyophilization of the plasma protein or after lyophilization. In a preferred embodiment of the invention, both the amino acids are added to a plasma protein solution, followed by lyophilization and the subsequent heat treatment of the lyophilizate.

Although both the amino acids may be removed after the dry heat treatment according to the invention, they should preferably be retained as they are in the plasma protein preparation as obtained.

Generally, the heat treatment is carried out at a temperature of about 30-100°C, preferably about 60°C, for a period of about 10 minutes to 200 hours, preferably about 10 hours to 100 hours.

The virus to be inactivated by the heat treatment in accordance with the invention includes those pathogenic viruses which are anticipated to be possible contaminants in human plasma proteins, in particular hepatitis viruses. G. Y. Rosenberg et al. [Proc. 12th Congr. Int. Soc. Blood Transf., Moscow 1969, Bibl. haemat., No.38, part II, pp. 474-478 (Karger, Basel, 1971)] report that dry heat treatment of a plasma protein with canine infectious hepatitis virus at 60°C for 10 hours resulted in complete hepatitis virus inactivation. The present inventors, too, subjected factor VIII, together with a variety of viruses, to dry heat treatment at 60°C and examined the heat-treated factor VIII for viral infectivity (cf. Table 3). The viral activity decreased with the lapse of time and, after 72 hours of heat treatment, there was no infectivity detected for any of the viruses tested.

The stability during heating can be increased by conducting the heat treatment according to the invention in an inert gas atmosphere. Said inert gas is, for example, nitrogen gas, argon or helium.

There is little correlation between the degree of purification of the plasma protein and the heat stability and accordingly, the stabilizing effect produced by both the amino acids will remain constant irrespective of the degree of purification of the plasma protein used. Therefore, the heat treatment according to the invention may be conducted in any step of the plasma protein purification process.

The dry state in which the dry heat treatment according to the invention is performed is a substantially anhydrous state, preferably a state in which the moisture content is as small as possible.  The moisture content is generally not more than 3%, preferably not more than 1%.

The method according to the invention is advantageous as an industrial method of preparing plasma protein preparations since possible contaminant viruses in said preparations can be inactivated without any great loss in the activity of plasma proteins which are precious blood preparations.

The following test examples and working examples are further illustrative of the present invention.  It is to be noted, however, that they are by no means limitative of the invention.

Test Example 1

A solution of blood coagulation factor VIII and a powdery lyophilizate of said solution were heat-treated on a water bath at 60°C for 20 hours and the changes in factor VIII activity were examined.  The results obtained are shown in Table 1.

Table 1

|  | Before heating | Heating at 60°C for 20 hours (without addition of any stabilizing agents) | |
|---|---|---|---|
|  |  | Solution | Lyophilizate powder |
| Blood coagulation factor VIII (activity) | 100% | 0% | 63.1% |

Test Example 2

Following addition of one of the stabilizing agents given in Table 2 at an addition level of 2% by weight to a blood coagulation factor VIII solution, said factor VIII solution was lyophilized. Then, the lyophilizate powder, in the dry state, was heat-treated in an autoclave at 121°C for 20 minutes and the residual factor VIII activity was determined by measuring the APTT by the one-stage Hardisty method. Each lyophilizate powder after the heat treatment was dissolved in distilled water and the solubility and clarity were observed. The results thus obtained are shown in Table 2.

The percent residual activity values in Table 2 are based on the activity before dry heat treatment which is taken as 100%.

Table 2

| Stabilizing agent (2%) | | Residual factor VIII activity (%) (Before heating: 100%) | Solubility[*1] | Clarity[*2] |
|---|---|---|---|---|
| No addition | | 0 | Δ | Δ |
| L-Lysine-L-glutamic acid mixture (1:1) | Added before lyophilization | 42.3 | O | O |
| Sorbitol | Ditto | . 0 | Δ | Δ |
| Sucrose | Ditto | 18.6 | Δ | Δ |
| Glycine | Ditto | 30.2 | Δ | Δ |
| L-Lysine L-glutamate | Ditto | 42.2 | O | O |
| Human albumin | Ditto | 13.3 | Δ | Δ |
| Mannitol | Ditto | 0 | Δ | Δ |
| Glucose | Ditto | 0 | Δ | Δ |
| Lactose | Ditto | 0 | Δ | Δ |
| L-Lysine | Ditto | 30 | Δ | Δ |
| L-Glutamic acid | Ditto | 19.5 | O | O |

Notes to Table 2:

    *1 Solubility:  O --- Immediate dissolution

                      Δ --- Difficult dissolution

    *2 Clarity:     O --- Colorless and transparent;

                            no insoluble matter

                      Δ --- Turbidity

From the above results, it is to be understood that plasma protein stabilization and solubility improvement can be attained only by the dry heat treatment performed under the combined use of an acidic amino acid and a basic amino acid.

Test Example 3

To a blood coagulation factor VIII solution was added L-lysine L-glutamate in an amount of 5 mg per 25 units, per milliliter, of factor VIII activity. After dissolution of the salt, a virus suspension in 0.05 M phosphate buffer (pH 7.1) was added. The mixture was stirred to attain homogeneity and lyophilized in a glass vial. After completion of the lyophilization, the vacuum was returned to ordinary pressure with nitrogen gas. The vial was stoppered tightly and immersed in a water bath maintained at 60°C, followed by heating at that temperature. Since 10-15 minutes was required for the vial inside temperature to reach 60°C, each heating time indicated in Table 3 was prolonged by 30 minutes.

The viral infectivity estimation was performed by the plaque forming method.

The results obtained are shown in Table 3.

Table 3

Inactivation of viruses intentionally added to
Factor VIII by dry-heating at 60°C for 0-72 hrs.

| Viruses | Viral infectivities * | | | |
|---|---|---|---|---|
|  | 0 hr | 10 hr | 24 hr | 72 hr |
| Vesicular stomatitis | $8.4 \times 10^3$ | 0 | 0 | 0 |
| Chikungunya | $2.8 \times 10^5$ | $4.5 \times 10$ | 0 | 0 |
| Vaccinia | $1.5 \times 10^3$ | $4.1 \times 10^2$ | $4.5 \times 10$ | 0 |
| Mumps | $4.0 \times 10^5$ | $7.0 \times 10^3$ | $4.0 \times 10$ | 0 |
| Herpes simplex | $4.5 \times 10^2$ | $1.0 \times 10^2$ | $7.5 \times 10$ | 0 |

* plaque forming units/ml

Example 1

To 1 g of purified factor VIII paste was added 10 ml of 0.02 M citrate-sodium chloride buffer (pH 6.8). After dissolution of the paste, both 0.05 g of L-lysine and 0.05 g of L-glutamic acid were added to the solution. After they were dissolved homogeneously, the solution was subjected to sterile filtration. The filtrate was distributed in glass ampuls in 1 ml portions, followed by a series of operations including lyophilization, filling the ampuls with nitrogen gas, fusing, and so on. The thus-obtained ampuls each containing a factor VIII lyophilizate

were heated on a water bath at 60°C for 72 hours.  Thereafter, 1 ml of distilled water was added to each ampul, and
the solubility and factor VIII activity were examined.  It
was thus found that the solubility was better in the L-
lysine-L-glutamic acid mixture-added group than in the no-
addition group.  In the former group, the recovery of
activity was not less than .90%.  The VIII R : Ag/VIII :
C ratio was 2.88, indicating no substantial denaturation due
to the heat treatment.

Example 2

    To 1 g of purified factor VIII paste was added 0.02 M
citrate-sodium chloride buffer (pH 6.8).  After dissolution,
the solution was subjected to sterile filtration and the
filtrate was lyophilized.  To the thus-obtained lyophilizate,
there were added, under aseptic conditions, both 0.05 g of
L-lysine and 0.05 g of L-glutamic acid, followed by mixing
to give a homogeneous mixture.  This dry sample was heated
on a water bath at 60°C for 72 hours.  The same procedure
was followed also in a control group in which neither L-
lysine nor L-glutamic acid was added.  Like in Example 1,
the solubility was better in the L-lysine-L-glutamic acid
mixture-added group than in the no-addition group, and the
recovery of activity in the former group was about 90%.

Example 3

    To a factor IX complex liquid bulk, there was added a
1:1 mixture of L-lysine and L-glutamic acid at an addition level of 1%, and the mixture was lyophilized.  The lyophilizate was tested under the same heat treatment conditions as in Example 1.  Separately, the factor IX complex liquid bulk was lyophilized in advance and then a 1:1 mixture of L-lysine and L-glutamic acid was added to the lyophilizate at an addition level of 1%, and the mixture was subjected to heat treatment in the same manner.  Furthermore, the same procedure was repeated in a control group without addition of L-lysine and L-glutamic acid.  Whereas, in the no addition group, the activity was reduced by about 20% or more as compared with the value before heat treatment, the activity reduction in either of the mixture-added groups was not more than 7%.  There was no difference observed between the addition-before-drying group and the addition-after-drying group.

Example 4

To a lyophilizate powder of fibrinogen, plasminogen or thrombin, there was added a 1:1 mixture of L-lysine and L-glutamic acid at an addition level of 0.5%, and the mixture was subjected to the same dry heat treatment as in Example 1.  In each case, there was obtained a dry-heat-treated preparation very good in solubility and clarity with a recovery of 90% or more in activity.

Example 5

To 1 g of purified factor VIII paste was added 10 ml of 0.02 M citrate-sodium chloride buffer (pH 6.8).  After dissolution, 0.1 g of L-lysine L-glutamate was added to the factor VIII solution.  After homogeneous dissolution of the salt, the solution was subjected to sterile filtration. The filtrate was distributed, in 1 ml portions, into glass ampuls.  The subsequent series of operations including lyophilization, sealing by fusion, and so on gave a factor VIII lyophilizate.  Thereafter, the ampul was heated on a water bath at 60°C for 72 hours.  After heating, 1 ml of distilled water was added to each ampul for dissolution of the contents.  The dissolution behavior was observed and the factor VIII activity was determined.  The L-lysine L-glutamate-added heat-treatment group showed better solubility than the no addition, no heat-treatment group and recovered almost 100% of the activity.  The VIII R : Ag/VIII : C ratio was 2.88, indicating that there had been no substantial denaturation due to the heat treatment.

Example 6

To a factor IX complex liquid bulk was added L-lysine L-glutamate at an addition level of 1% and the mixture was subjected to testing under the same heat treatment conditions as used in Example 5.  While the no-addition heat treatment group showed an activity reduction of about 20% as compared with the value before heat treatment, the activity reduction in the salt-added heat treatment group was not more than 7%.

Example 7

To a solution of fibrinogen, plasminogen or thrombin, there was added L-lysine L-glutamate at an addition level of 0.5%, followed by the same dry heat treatment as Example 5. In each case, there was obtained a dry heat-treated preparation very good in solubility and clarity with a potency recovery of not less than 90%. Each preparation was thus comparable to the preparation before dry heat treatment.

Claims

1.   A method of heat-treating plasma proteins which comprises heating a virus-contaminated plasma protein in a dry state and in the presence of both an acidic amino acid and a basic amino acid until virus inactivation is complete.

2.   The method of Claim 1, wherein the acidic amino acid and basic amino acid form a salt.

3.   The method of Claim 1 or 2, wherein the combination of the acidic amino acid and basic amino acid is the combination of L-glutamic aicd and L-lysine.

4.   The method of Claim 1 or 2, wherein the combination of the acidic amino acid and basic amino acid is the combination of aspartic acid and lysine.

5.   The method of any of claims 1 - 4, wherein the plasma protein is selected from blood coagulation factor II, blood coagulation factor V, blood coagulation factor VII, blood coagulation factor VIII, blood coagulation factor IX, blood coagulation factor X.

6.   The method of any of claims 1 - 4, wherein the plasma protein is selected from fibrinogen, plasminogen, and thrombin.

7.   The method of any of claims 1 - 6, wherein the virus is a hepatitis virus.

8.   The method of any of claims 1 - 7, wherein the heating is conducted in an inert gas atmosphere.